# EUROPEAN PATENT SPECIFICATION

(11) **EP 1 618 114 B1**
(45) Date of publication and mention of the grant of the patent: **14.07.2010**
(21) Application number: 04727526.8
(22) Date of filing: 15.04.2004
(51) Int. Cl.: C07D 501/06, C07D 501/44

(54) **PROCESSES FOR THE PREPARATIONS OF CEFEPIME**
VERFAHREN ZUR HERSTELLUNG VON CEFEPIM
PROCEDES DE PREPARATION DE CEFEPIME

(30) Priority: 16.04.2003 AT 5862003; 16.04.2003 AT 5852003; 16.04.2003 AT 5842003
(43) Date of publication of application: 25.01.2006
(60) Divisional application: 10005006.1
(73) Proprietor: SANDOZ AG, 4056 Basel (CH)
(72) Inventor: LUDESCHER, Johannes, A-6252 Breitenbach (AT); STURM, Hubert, A-6020 Innsbruck (AT); WOLF, Siegfried, A-6230 Brixlegg (AT)
(74) Representative: Wichmann, Hendrik
(86) International application number: PCT/EP2004/003988
(87) International publication number: WO 2004/092183

(56) References cited:
- EP-A- 0 137 440
- EP-A- 0 531 981
- US-A- 4 489 072
- US-A- 5 574 154
- US-A1- 2002 156 272
- US-B1- 6 384 215
- DATABASE CASREACT XP002297198 Database accession no. 140:111151 & GONG, PING; ZHAO, YANFANG; FENG, RUNLIANG; ZHANG, ZHANTAO: HONGGUO YAOWU HUAXUE ZAZHI, vol. 12, no. 6, 2002, pages 350-351,362,
- WALKER D G: "NEW CEPHALOSPORIN ACYLATING AGENTS DERIVED FROM SYN-2-(2-AMINOTHIAZOL-4-YL)-2-METHOXYIMINO ACETIC ACID. APPLICATION TO THE SYNTHESIS OF CEFEPIME SULFATE" TETRAHEDRON LETTERS, ELSEVIER SCIENCE PUBLISHERS, AMSTERDAM, NL, vol. 31, no. 45, 1990, pages 6481-6484, XP002035970 ISSN: 0040-4039

## Description

The present invention relates to the preparation of 1-[[(6R,7R)-7-[[(2Z)-(2-amino-4-thiazolyl)methoxyimino)acetyl]amino]-2-carboxy-8-oxo-5-thia-1-azabicyclo[4.2.0]oct-2-en-3-yl]methyl]-1-methyl-pyrrolidinium dihydrochloride hydrate (cefepime dihydrochloride monohydrate), hereafter "cefepime". Cefepime is a valuable 4^{th} generation injectable cephalosporin with antibacterial properties, see e.g. The Merck Index Thirteenth Edition, Item 1935, and is used e.g. in the form of a dihydrochloride hydrate of formula I

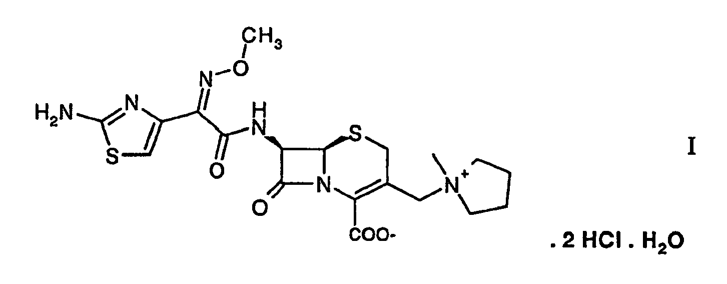

Presently known methods for preparing cefepime are far from straightforward. For example, it is known that the 7-acyl side chain as the difficultly obtainable 2-(2-aminothiazol-4-yl)-2-methoxyimino-acetic acid chloride hydrochloride must be used for the production of cefepime, in order to obtain an active ingredient which is pure in respect of the by-products known as anti-isomer and Δ-2 isomer.

The present applicants have sought to overcome the problems of hitherto known processes. Gong P. et al. Chinese Journal of Medicinal Chemistry, Vol 12, 2002, 350-351 discloses the preparation of compound I in dichloromethane.

Unless otherwise stated, alkyl means C₁-C₈ alkyl, e.g. C₁-C₄ alkyl, e.g. methyl, ethyl, propyl or butyl and may be straight or branched chain.

Unless otherwise stated, the compounds of formula IIA and IIB are referred to as compounds of formula II.

It will be appreciated that the compound of formula II may exist in mixtures. Thus the compound of formula IIA may exist in a mixture having a proportion where n is 1, and a proportion where n is 2. 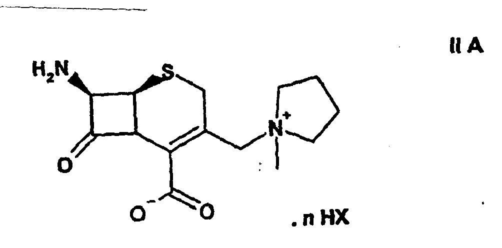 wherein
n is 0-2 and
X is chloride, bromide or iodide.

The compounds of formula II may be used in free base form, as a mono-addition salt or as a di-addition salt with a hydrohalic acid such as hydrochloric acid, hydrobromic acid or hydriodic acid. The addition salts may additionally be present in solvated form, e.g. as a hydrate.

This invention provides a novel process for the production of cefepime which is notable for the simplicity of the choice of solvent and the accessibility and facile handling of the 7-acyl side chain, and which at the same time leads to an active ingredient with high purity in respect of the above-mentioned by-products.

The process comprises the reaction of a pyrrolidinium-1-[(7-amino-2-carboxy-8-oxo-5-thia-1-azabicyclo[4.2.0]oct-2-en-yl)methyl]-halide, an acid addition salt thereof or its free base of formula IIA with (Z)-(2-aminothiazol-4-yl)methoxyimino-acetic acid-2-mercaptobenzothiazolylester of formula XII 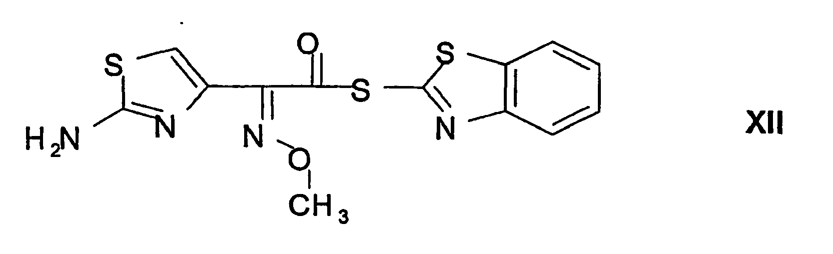 in an acetonic or aqueous/acetonic solution, optionally in the presence of a base, wherein cefepime dihydrochloride monohydrate is precipitated in crystalline form directly from the reaction mixture by adding HCl.

The process is straightforward. Neither extraction steps nor more complex purification operations are necessary. The solvent regeneration is an especially simple procedure, in that only one solvent is used both for the acylation reaction and for the crystallisation step.
The intermediate compound of formula IIA may be present as mono- addition salt or di-addition salt or a mixture thereof. In addition, the intermediate of formula IIA may be present in the form of a solvate, for example a hydrate. The usual addition salts are represented by the mono- and dihydrochloride or the hydriodide.

Depending on the salt form, the corresponding acid addition salt is released for the reaction with the acylation agent with the assistance of the necessary amount of a base, preferably a trialkylamine. Accordingly, a mono-addition salt is released with approximately one molar equivalent of base, and a di-addition salt is correspondingly released with approximately two. However, it is also possible to react the corresponding acid addition salt with (Z)-(2-aminothiazol-4-yl)methoxyimino-acetic acid-2-mercapto-benzothiazolylester without adding a base.

If the intermediate of formula IIA is used as the mono- or dihydrochloride, the active ingredient cefepime is obtained as the pure dihydrochloride. If the intermediate is used as the hydriodide, the recrystallised product is practically and substantially free from traces of iodide.

Alternatively, foreign ions can be removed from the reaction solutions by known methods, for example with the assistance of an anion exchanger.

Suitable trialkylamines are C₁-C₈-triakylamines, for example triethylamine or tributylamine. The presence of water in the acylation reaction in principle also allows the use of inorganic bases, for example sodium or potassium hydroxide or an alkali hydrogen carbonate or alkali carbonate, e.g. sodium or potassium hydrogen carbonate or carbonate.

The reaction is preferably carried out in the presence of water: the amount of water is not critical; there must be balanced solubility of the cephalosporin intermediate of formula IIA and of the active ester of formula XII. The water/acetone ratio may be between 1:10 to 10:1, and preferably a water/acetone ratio of 1:1 to 1:5 is used for the acylation reaction. After the acylation reaction, in order to crystallise cefipime dihydrochloride, hydrochloric acid is added, preferably aqueous concentrated hydrochloric acid, and a pH value of less than 3, preferably less than 1, is set. By adding acetone, the crystallisation of cefepime dihydrochloride is then completed. Preferred water/acetone ratios in the crystallisation step are ratios of 1:1 to 1:20, especially ratios of 1:3 to 1:10.

The processes of this invention may be carried out between -40°C and room temperature, for example between -35°C and 15°C, preferably between -25°C and about 1°C:

Following is a description by way of example only of the process of this invention.

The following abbreviations are used:
- NMP⁺: to denote N-methylpyrrolidinium
- NMP-ACA: to denote an intermediate compound of Formula IIA

### Example 1

### Preparation of 1-[[(6R,7R)-7-[[(2Z)-(2-amino-4-thiazolyl)methoxyimino)acetyl]amino]-2-carboxy-8-oxo-5-thia-1-azabicyclo[4.2.0]oct-2-en-3-yl]methyl]-1-methyl-pyrrolidinium dihydrochloride hydrate

44.3 g of pyrrolidinium-1-[(7-amino-2-carboxy 8-oxo-5-thia-1-azabicyclo[4.2.0]oct-2-en-yl)methyl]-iodide monohydrate ( NMP-ACA) are suspended in a mixture of 200 ml of H₂O and 400 ml of acetone. 38.5 g of (Z)-(2-aminothiazol-4-yl)methoxy-iminoacetic acid-2-mercaptobenzothiazolylester are added to the suspension. At a temperature of ca. 15°, a mixture of 13.9 ml of triethylamine and 14 ml of acetone is slowly added dropwiseto the suspension over the course of 3 hours. The resulting cloudy solution is stirred for a total of 6.5 hours at 20°C.
33 ml of 37% HCl are subsequently added to the reaction mixture, and then ca. 300 ml of acetone are added whilst stirring. The mixture is seeded with seed crystals, and within ca. 90 minutes a suspension is produced. Subsequently, within 90 minutes, 1700 ml of acetone are added dropwise whilst stirring gently. The suspension is stirred for a further one hour at room temperature, and then the title compound is isolated through a suction filter, and the product is washed with 250 ml of acetone/H₂O mixture (90/10) and with a total of 500 ml of acetone in two portions. The product is subsequently dried for ca. 18 hours at room temperature in a vacuum drying chamber.
Yield 51.8 g

### purity: HPLC: 98.8 area percent

### Example 2

### Recrystallisation of 1-[[(6R,7R)-7-[[(2Z)-(2-amino-4-thiazolyl)methoxyimino)acetyl]amino]-2-carboxy-8-oxo-5-thia-1-azabicyclo[4.2.0]oct-2-en-3-yl]methyl]-1-methyl-pyrrolidinium dihydrochloride hydrate

50.0 g of cefepime dihydrochloride hydrate are dissolved in 200 ml of H₂O. 22 ml of 6 n HCl are added and then the solution is mixed with 5 g of activated carbon. The suspension is stirred for 10 minutes at room temperature and then filtered through a suction filter. The filter layer is then washed with 50 ml of H₂O, and the combined filtrates are mixed with 600 ml of acetone until turbidity occurs. The resulting suspension is stirred for 15 minutes and then a further 1400 ml of acetone are added over the course of one hour whilst stirring gently. The suspension is stirred for another one hour at room temperature, and the product is subsequently isolated through a suction filter. The product is washed with a total of 500 ml of acetone and dried for ca. 18 hours at room temperature in a vacuum drying chamber.
Yield 45.01 g
purity HPLC: 99.7 area percent

## Claims

1. A process for producing the compound of formula I 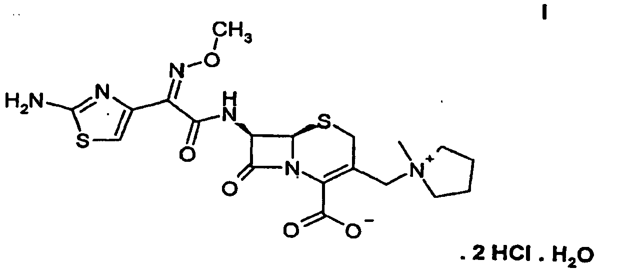 **characterised in that** a compound of formula IIA, 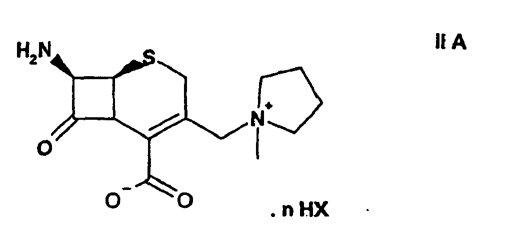 wherein n is 0-2 and X is chloride, bromide or iodide in unsolvated or solvated form, is reacted optionally after addition of a base, with a compound of formula XII 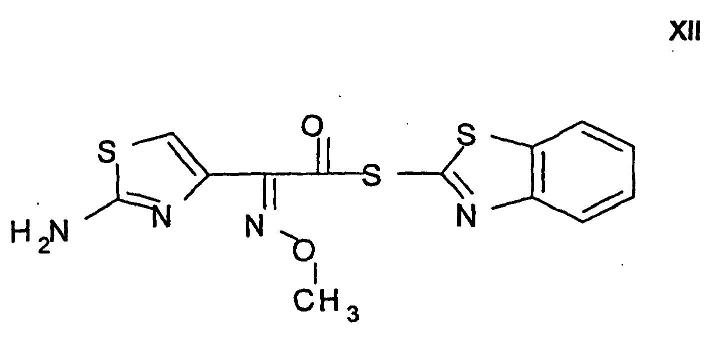 in acetone or aqueous acetone, and the compound of formula I precipitated in crystalline form from the reaction mixture by adding HCl.

2. A process as claimed in claim 1, wherein pyrrolidinium-1-[(7-amino-2-carboxy-8-oxo-5-thia-1-azabicyclo[4.2.0]oct-2-en-yl)methyl]-iodide monohydrate is used.

3. A process as claimed in claim 1, wherein pyrrolidinium-1-[(7-amino-2-carboxy-8-oxo-5-thia-1-azabicyclo[4.2.0]oct-2-en-yl)methyl]-chloride is used, optionally in solvated form.

4. A process as claimed in claim 1 wherein pyrrolidinium-1-[(7-amino-2-carboxy-8-oxo-5-thia-1-azabicyclo[4.2.0]oct-2-en-yl)methyl]-dihydrochloride is used, optionally in solvated form.

5. A process as claimed in any one of claims 1 to 4, wherein a C₁-C₈-trialkylamine, KOH or NaOH, or an alkali hydrogen carbonate or potassium carbonate, is used as the base.

## Patentansprüche

1. Verfahren zum Herstellen der Verbindung nach Formel I 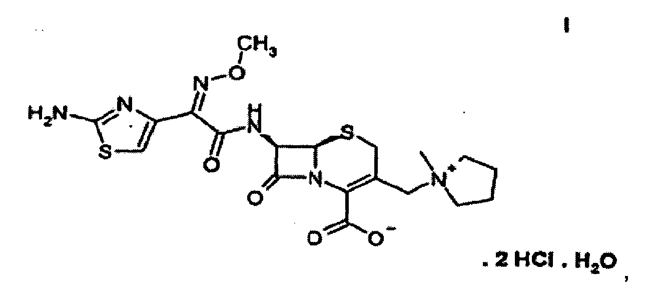 **dadurch gekennzeichnet, dass** man eine Verbindung der Formel IIA, 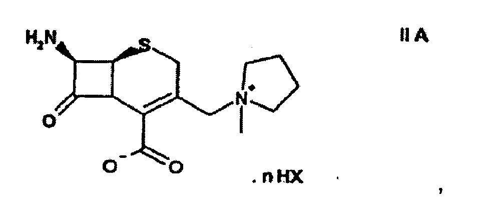 wobei n 0-2 ist und X Chlorid, Bromid oder Jodid ist, in unsolvatisierter oder solvatisierter Form, gegebenenfalls nach Zugabe einer Base mit einer Verbindung der Formel XII 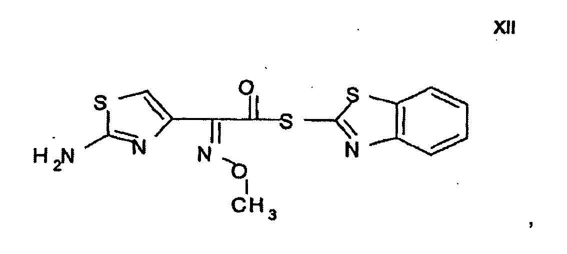 in Aceton oder wässrigem Aceton umsetzt, und die Verbindung der Formel I in kristalliner Form aus dem Reaktionsgemisch durch Zugabe von HCl präzipitiert.

2. Verfahren wie in Anspruch 1 beansprucht, wobei Pyrrolidinium-1-[(7-amino-2-carboxy-8-oxo-5-thia-1-azabicyclo[4.2.0]oct-2-en-yl)methyl] jodid Monohydrat verwendet wird.

3. Verfahren, wie in Anspruch 1 beansprucht, wobei Pyrrolidinium-1-[(7-amino-2-carboxy-8-oxo-5-thia-1-azabicyclo [4.2.0] oct-2-en-yl)methyl]-chlorid, gegebenenfalls in solvatisierter Form, verwendet wird.

4. Verfahren, wie in Anspruch 1 beansprucht, wobei Pyrrolidinium-1-[(7-amino-2-carboxy-8-oxo-5-thia-1-azabicyclo[4.2.0]oct-2-en-yl)methyl]-dihydrochlorid, gegebenenfalls in solvatisierter Form, verwendet wird.

5. Verfahren, wie in einem beliebigen der Ansprüche 1 bis 4 beansprucht, wobei ein C₁-C₈-Trialkylamin, KOH oder NaOH oder ein Alkalihydrogencarbonat oder Kaliumcarbonat als Base verwendet wird.

## Revendications

1. Procédé de production du composé de formule I 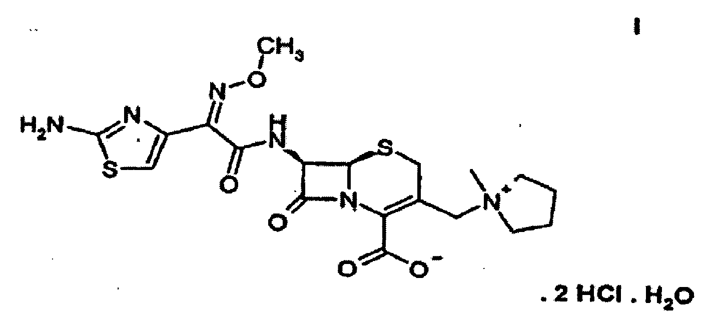 **caractérisé en ce qu'**un composé de formule IIA, 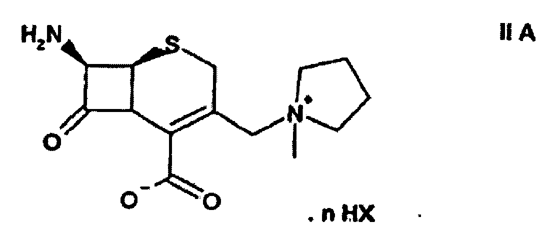 dans laquelle n vaut de 0 à 2 et X est le chlore, le brome ou l'iode, sous forme non solvatée ou solvatée, est mis à réagir facultativement après ajout d'une base, avec un composé de formule XII 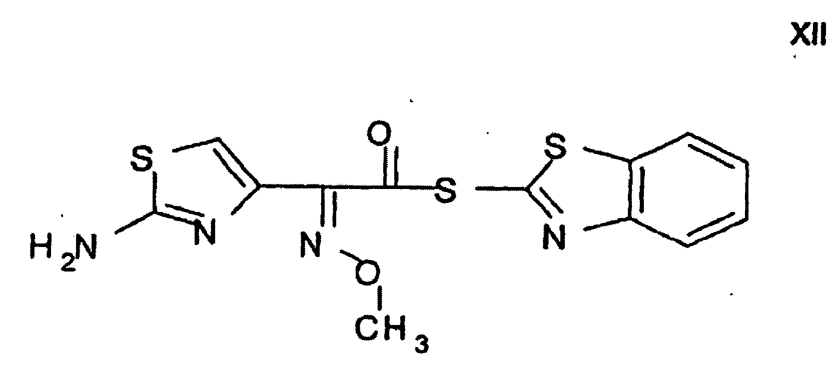 dans de l'acétone ou de l'acétone aqueuse, et que le composé de formule I est précipité sous forme cristalline à partir du mélange réactionnel en ajoutant du HCl.

2. Procédé selon la revendication 1, dans lequel de l'iodure de pyrrolidinium-1-[(7-amino-2-carboxy-8-oxo-5-thia-1-azabicyclo[4.2.0]oct-2-én-yl)méthyle] monohydraté est utilisé.

3. Procédé selon la revendication 1, dans lequel du chlorure de pyrrolidinium-1-[(7-amino-2-carboxy-8-oxo-5-thia-1-azabicyclo[4.2.0]oct-2-én-yl)méthyle] est utilisé, facultativement sous forme solvatée.

4. Procédé selon la revendication 1, dans lequel du dichlorhydrate de pyrrolidinium-1-[(7-amino-2-carboxy-8-oxo-5-thia-1-azabicyclo-[4.2.0]oct-2-én-yl)méthyle] est utilisé, facultativement sous forme solvatée.

5. Procédé selon l'une quelconque des revendications 1 à 4, dans lequel une trialkylamine en C₁-C₈, du KOH ou du NaOH, ou un hydrogénocarbonate alcalin ou du carbonate de potassium, est utilisé comme base.
